# FASCICULE DE BREVET EUROPEEN

(11) **EP 0 732 910 B1**
(45) Date de publication et mention de la délivrance du brevet: **11.11.1998**
(21) Numéro de dépôt: 95903393.7
(22) Date de dépôt: 12.12.1994
(51) Int. Cl.: A61F 13/15

(54) **ARTICLE D'HYGIENE ABSORBANT JETABLE AVEC VOILE DE SURFACE POURVU D'UNE OUVERTURE POUR RECEVOIR LES SELLES**
WEGWERFBARER ABSORBIERENDER HYGIENEARTIKEL MIT EINER EINE STUHLAUFNEHMENDEN ÖFFNUNG AUFWEISENDEN OBERFLÄCHENLAGE
DISPOSABLE ABSORBENT HYGIENE ARTICLE HAVING A SURFACE WEB WITH AN APERTURE FOR RECEIVING STOOLS

(30) Priorité: 13.12.1993 FR 9314926
(43) Date de publication de la demande: 25.09.1996
(73) Titulaire: SCA Mölnlycke, 59497 Linselles Cédex (FR)
(72) Inventeur: DURANT, Bénédicte, F-59280 Armentières (FR); SVERNLOV, Anna, S-430 41 Kullavik (SE)
(74) Mandataire: Casalonga, Axel
(86) Numéro de dépôt international: FR9401450
(87) Numéro de publication internationale: WO9516417

(56) Documents cités:
- EP-A- 0 357 298
- EP-A- 0 359 410
- EP-A- 0 374 640
- EP-A- 0 508 477
- WO-A-93/12748
- GB-A- 2 161 059

## Description

L'invention concerne, d'une manière générale, un article d'hygiène absorbant jetable, tel qu'une couche-culotte absorbante jetable destinée aux enfants en bas âge ou aux personnes incontinentes, du type comprenant un coussin absorbant disposé entre une feuille extérieur de support imperméable aux liquides et une feuille ou voile de surface intérieur perméable aux liquides.

Plus particulièrement, l'invention concerne un tel article d'hygiène absorbant jetable dont la feuille de surface intérieure comporte une ouverture destinée à recevoir les selles et des petites barrières latérales, longitudinales, situées en avant de l'ouverture pour empêcher des fuites d'urine.

Un tel article est connu du document WO-A-93/12748, qui correspond au préambule de la revendication 1.

Des articles d'hygiène de ce type sont connus par exemple par le brevet US-A-4 662 877 (Johnson & Johnson). Suivant ce document, la feuille de surface intérieure de l'article d'hygiène est pourvue d'une ouverture de forme allongée, décalée vers l'avant de l'article, dont la longueur est choisie de sorte que les selles soient normalement dirigées à travers cette ouverture sur le centre de l'article au-dessus du coussin absorbant. Les bords longitudinaux de l'ouverture sont pourvus d'éléments élastiques fixés à l'état tendu.

La demande de brevet français FR-A- 92 10601 (Peaudouce) décrit également une couche-culotte dont la feuille de surface intérieure comporte une ouverture allongée centrale et deux doubles plis longitudinaux élastifiés, s'étendant sur toute la longueur de la couche-culotte, situés de part et d'autre de l'ouverture centrale. Les deux replis de chaque double plis sont solidarisés l'un à l'autre sur toute la longueur des doubles plis.

La demande de brevet EP-A- 357 298 (The Procter and Gamble company) décrit un article absorbant jetable tel qu'une couche-culotte, comportant une feuille extérieure de support imperméable aux liquides, une feuille perméable à l'urine, et un coussin absorbant entre la feuille extérieure de support et la feuille perméable à l'urine. La feuille perméable à l'urine comporte un passage qui permet la communication des matières fécales solides avec le coussin, isolant ainsi ces matières fécales de la peau de l'utilisateur. Ce passage est une ouverture oblongue légèrement décalée vers l'arrière de l'article. Des éléments élastiques sont fixés à la feuille perméable à l'urine en avant et en arrière de l'ouverture oblongue pour assurer une contraction de la feuille perméable à l'urine dans ces zones.

La demande de brevet européen EP-A-374 640 (The Procter & Gamble Company) décrit un article absorbant comprenant une feuille extérieure de support imperméable aux liquides, une feuille de surface perméable aux liquides et un coussin absorbant disposé entre la feuille extérieure de support et la feuille de surface. Une barrière est disposée de façon adjacente à chaque bord longitudinal de l'article et présente un bord proximal et un bord distal. Des moyens d'écartement, tels que des éléments élastiques, écartent le bord distal des barrières de la feuille de surface. Les barrières sont fixées dans une première région de ceinture de telle sorte que leurs bords distaux soient à l'intérieur par rapport à leurs bords proximaux et dans une seconde région de ceinture de telle sorte que leurs bords distaux soient à l'extérieur par rapport à leurs bords proximaux. Ainsi, les barrières sont infléchies et présentent une partie repliée vers l'extérieur pour assurer une action d'étanchéité autour des fesses de l'utilisateur et contenir les exsudats et une partie relevée réalisant un canal pour contenir, confiner et maintenir les exsudats corporels.

Les demandes de brevets britanniques GB-A-2 265 834, 2 266 055 et 2 265 550 (Uni-charm) décrivent également des couche-culottes dont la feuille de surface intérieure comporte une ouverture centrale de forme allongée, cette ouverture centrale pouvant être munie de deux volets longitudinaux latéraux dont un bord est réuni aux bords longitudinaux de l'ouverture et dont l'autre bord est libre, les bords de ces volets pouvant être élastifiés.

Bien que ces dispositifs décrits dans l'art antérieur s'avèrent relativement efficaces, il serait encore souhaitable d'améliorer le confinement et l'isolation des matières fécales, en particulier de l'urine, éviter un contact prolongé de ces matières fécales avec la peau de l'utilisateur, et également améliorer l'étanchéité vis-à-vis de l'émission d'urine.

L'invention a donc pour but de fournir un article d'hygiène absorbant jetable qui présente à la fois une meilleure isolation des matières fécales, en particulier vis-à-vis de l'émission d'urine, et une étanchéité améliorée vis-à-vis de l'émission d'urine.

Selon l'invention, on réalise un article d'hygiène absorbant jetable, tel qu'une couche-culotte, symétrique par rapport à un axe longitudinal médian, ayant des bords transversaux avant et arrière, et qui comprend une feuille extérieure de support imperméable aux liquides, de forme générale rectangulaire, un coussin absorbant de forme générale rectangulaire, fixé sur ladite feuille extérieure de support et une feuille de surface intérieure perméable aux liquides recouvrant le coussin absorbant. Cette feuille de surface intérieure perméable aux liquides est fixée sur son pourtour à la feuille extérieure de support.

L'article d'hygiène comprend, en outre, des moyens d'attache pour permettre sa fermeture autour de la taille d'un utilisateur.

L'article comprend, par ailleurs, des éléments élastiques longitudinaux fixés à l'état tendu à la feuille de support à l'extérieur des bords longitudinaux du coussin absorbant, au moins dans une zone d'entrejambes.

La feuille de surface intérieure perméable aux liquides est pourvue d'au moins une ouverture longitudinale, symétrique par rapport à l'axe longitudinal médian de la couche-culotte, généralement de forme oblongue ou allongée, et de largeur inférieure à celle du coussin absorbant. Selon l'invention, cette ouverture longitudinale est décalée par rapport au centre de l'article, en direction du bord transversal arrière de la couche-culotte afin d'être disposée de façon symétrique par rapport à un point d'acquisition des selles situé à une distance prédéterminée du bord transversal arrière de l'article. La feuille de surface intérieure est également fixée au coussin absorbant sauf dans une zone située en dessous de l'ouverture, qui, de préférence, est de dimension supérieure à l'ouverture.

La feuille de surface intérieure comprend également, selon l'invention, deux plis longitudinaux formant des gaines tubulaires fermées, sensiblement parallèles à l'axe longitudinal médian de l'article, qui s'étendent sur toute la longueur de l'article, de part et d'autre de l'ouverture longitudinale. Au moins un élément élastique est fixé, à l'état tendu, dans la partie supérieure des gaines formées par les plis. Chacun des plis comprend un première zone longitudinale allant du bord transversal arrière de l'article sensiblement jusqu'au bord transversal avant de l'ouverture longitudinale dans laquelle le pli est rabattu vers l'intérieur en direction de l'axe longitudinal médian et est fixé à la partie sous jacente de la feuille de surface intérieure sur toute la longueur de cette première zone, par exemple par une ligne de collage. Chacun des plis comprend également une seconde zone allant sensiblement du bord transversal avant de l'ouverture longitudinale jusqu'au bord transversal avant de la couche-culotte dans laquelle le pli est libre sur une majeure partie de cette seconde zone, est rabattu soit vers l'extérieur dans la direction opposée à l'axe longitudinal médian soit vers l'intérieur en direction dudit axe et est fixé à la partie sous-jacente de la feuille de surface intérieure à proximité du bord transversal avant de l'article, par exemple par collage.

Du fait que les plis, dans leurs premières zones, sont rabattus vers l'intérieur et fixés à la partie sous-jacente de la feuille de surface, on obtient une meilleure adaptation de l'ouverture longitudinale à l'anatomie de l'utilisateur, en particulier au niveau du point d'acquisition des selles, puisque l'ouverture est centrée sur ce point. En effet, ces premières zones élastifiées des plis, qui sont fixées aux parties sous-jacentes de la feuille de surface le long des bords longitudinaux de l'ouverture, soulèvent la feuille de surface le long des bords longitudinaux de l'ouverture et l'appliquent aux fesses de l'utilisateur, quelque soit l'attitude de l'utilisateur. D'autre part, comme les plis sont fixés dans leurs secondes zones aux parties sous-jacentes respectives de la feuille de surface, uniquement à proximité du bord transversal avant de l'article on forme des barrières latérales d'étanchéité qui assurent une meilleure étanchéité aux risques de fuites d'urine transversales.

Dans une réalisation recommandée les plis longitudinaux sont rabattus vers l'extérieur par rapport à l'axe longitudinal médian, ce qui représente une torsion de 180° par rapport aux premières zones des plis, les petites barrières ainsi formées se relèvent en ayant tendance à se tourner vers l'extérieur et en s'écartant l'une de l'autre en direction du bord transversal avant de l'article ce qui assure une meilleure adaptation des barrières à l'anatomie de l'utilisateur et offre une surface maximale d'absorption des urines dans la partie avant de l'article quelque soit l'attitude de l'utilisateur.

Dans une autre réalisation recommandée, l'article d'hygiène absorbant comporte en outre une feuille intermédiaire, perméable aux liquides, située entre le coussin absorbant et la feuille de surface intérieure, cette feuille intermédiaire recouvrant le coussin au moins dans la zone de l'ouverture longitudinale et étant fixée au coussin absorbant. De préférence encore, cette feuille intermédiaire, fixée au coussin, s'étend sur toute la longueur de l'article d'hygiène absorbant et a une largeur supérieure à celle de l'ouverture longitudinale. La feuille de surface intérieure peut alors être fixée à la feuille intermédiaire, à l'exception, comme précédemment d'une zone située sous l'ouverture.

Afin d'encore améliorer l'efficacité de l'ouverture longitudinale, des éléments élastiques, de préférence parallèles, sont fixés à l'état tendu, symétriquement par rapport à l'axe longitudinal médian de l'article, à la feuille de surface intérieure, en avant de l'ouverture longitudinale. De préférence encore, ces éléments élastiques sont disposés de telle sorte qu'ils viennent s'arrêter près du bord transversal avant de l'ouverture, de façon à maintenir la surface de l'ouverture la plus grande possible et à soulever la feuille de surface dans cette zone au-dessus du coussin absorbant.

Un autre mode particulier de réalisation selon l'invention est indiqué dans la revendication 5.

La suite de la description se réfère aux figures annexées qui représentent, respectivement :
figure 1, une vue de dessus, en partie arrachée, d'une couche-culotte selon la présente invention;
figure 2, une vue en coupe faite suivant la ligne II-II de la figure 1;
figure 3, une vue en coupe faite suivant la ligne III-III de la figure 1;
figure 4, une vue en coupe faite suivant la linge IV-IV de la figure 1; et
figure 5, une vue de dessus, en partie arrachée, d'une autre réalisation d'une couche-culotte selon l'invention.

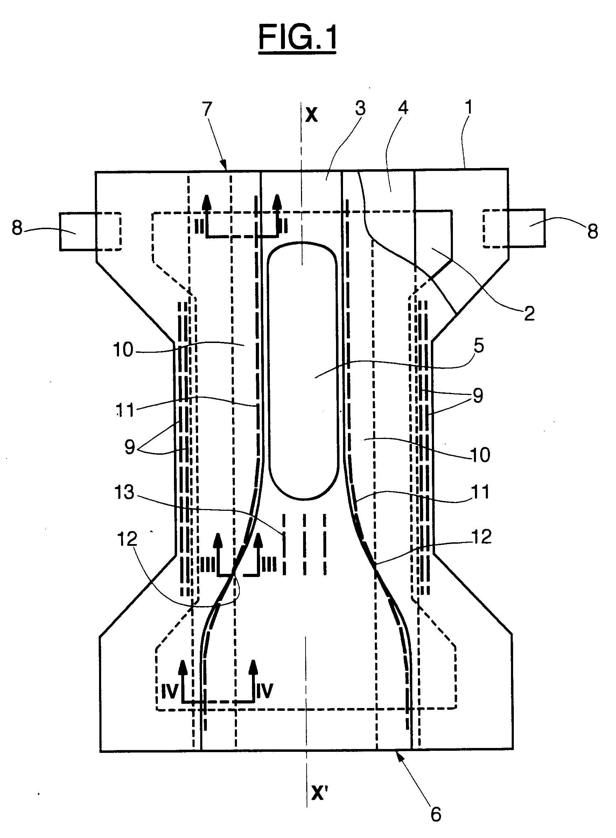

En se référant aux figures 1 à 4, la couche-culotte représentée comprend, de façon connue en soit, une feuille de support 1 imperméable aux liquides, un coussin absorbant 2, par exemple en pâte fluff de cellulose, éventuellement avec incorporation de matière polymère dite super absorbante, et une feuille de surface 3 perméable aux liquides, par exemple en voile de non-tissé hydrophile. Les deux feuilles 1 et 3 ont les mêmes dimensions et la même forme en sablier, c'est-à-dire une forme rectangulaire avec deux échancrures latérales opposées délimitant dans le sens de la longueur de la coche-culotte, une zone d'entrejambes de largeur réduite entre deux zones d'extrémité de largeur accrue dont les bords transversaux définissent des bords transversaux avant 6 et arrière 7 de la couche-culotte.

Le coussin absorbant 2 disposé entre les feuilles 1 et 3 présente également une forme en sablier, mais de dimension plus faible que les deux feuilles 1 et 3 qui sont reliées entre elles, par exemple par collage, sur le pourtour du coussin 2. Des éléments élastiques 9 longitudinaux constitués, par exemple, chacun par un ou plusieurs brins ou fils élastiques ou par une bandelette élastique, sont fixés à l'état tendu à la feuille de support 1, au moins dans la zone d'entrejambes, entre le bord longitudinal de la feuille 1 et le bord longitudinal correspondant du coussin absorbant 2.

Comme le montre la figure 1, la feuille de surface intérieure 3 comporte une ouverture, généralement de forme oblongue ou allongée, symétrique par rapport à l'axe longitudinal médian XX' de la couche-culotte et de largeur inférieure à la largeur du coussin absorbant dans la zone d'entrejambes. De préférence, cette ouverture a une largeur inférieure à la moitié de la largeur du coussin 2 dans la zone d'entrejambes. Cette ouverture de forme allongée est décalée par rapport au centre de la couche-culotte, en direction du bord transversal arrière 7 de la couche-culotte, de façon à être disposée symétriquement par rapport à un point d'acquisition des selles situé à une distance prédéterminée du bord transversal arrière 7 de la couche-culotte. Bien évidemment les dimensions de cette ouverture 5 dépendent de l'utilisateur auquel est destinée la couche-culotte. Dans une réalisation, donnée à titre d'exemple pour une couche-culotte de la taille dite "MAXI" (8-18 kg), l'ouverture 5 est sensiblement rectangulaire ayant une longueur de 200 mm et une largeur de 40 mm, et est disposée de façon symétrique par rapport au point d'acquisition des selles qui dans cet exemple se situe à une distance de 125 mm du bord transversal arrière du coussin absorbant. De préférence, la couche-culotte comporte une bande ou feuille intermédiaire 4 perméable aux liquides, par exemple en non-tissé hydrophile, s'étendant sur toute la longueur de la couche-culotte et de largeur supérieure à la largeur du coussin absorbant dans la zone d'entrejambes et, donc également à la largeur de l'ouverture longitudinale 5. De plus, la longueur de cette feuille intermédiaire 4 pourrait également être inférieure à celle de la couche-culotte, et même à celle du coussin absorbant pour autant qu'elle soit supérieure à la longueur de l'ouverture longitudinale 5. De préférence, cette feuille intermédiaire est fixée au coussin absorbant 2. La feuille de surface intérieure 3 est fixée à cette feuille intermédiaire 4 à l'exception d'une zone située en dessous de l'ouverture longitudinale 5. Cette zone dans laquelle la feuille de surface intérieure 3 n'est pas fixée à la feuille intermédiaire 4 est de dimension supérieure à l'ouverture longitudinale 5. Par exemple, pour les dimensions de l'ouverture longitudinale 5 données précédemment, cette zone peut être une zone rectangulaire de 370 mm de longueur sur 100 mm de large.

La feuille de surface intérieure 3 est également pourvue dans sa partie avant d'éléments élastiques longitudinaux 13, parallèles, disposés à l'état tendu, sensiblement parallèlement à l'axe longitudinal médian XX' de la couche-culotte. Ces éléments élastiques s'arrêtent de préférence près du bord transversal avant de l'ouverture 5, de façon à maintenir la surface de l'ouverture la plus grande possible et à soulever la feuille de surface dans cette zone au-dessus du coussin absorbant. Bien qu'on ait représenté trois éléments élastiques 13, il est bien évident qu'on peut en utiliser un nombre supérieur ou inférieur, par exemple, seulement deux éléments élastiques disposés symétriquement de part et d'autre de l'axe longitudinal médian XX', écartés d'une distance appropriée pour ne pas gêner le passage de l'urine vers le coussin 2 absorbant dans la partie centrale de la couche-culotte.

D'autres orientations qu'une orientation parallèle à l'axe longitudinal médian peuvent aussi être utilisées pour ces éléments élastiques 13, en particulier une disposition inclinée par rapport à l'axe longitudinal médian, ayant la forme générale d'un V, avec la pointe du V dirigée vers le bord avant de l'ouverture 5.

Les éléments élastiques 13 peuvent être réalisés en tout matériau ayant des propriétés élastiques suffisantes, en particulier des fils de caoutchouc ou de Lycra® ou en matériau thermoélastifiable.

De préférence, les éléments élastiques 13 sont fixés à la feuille de surface 3 par l'intermédiaire d'une feuille supplémentaire (non représentée), telle qu'un non tissé, au moyen de tout procédé approprié tel que collage, soudure aux ultrasons ou thermoscellage, de sorte que ces éléments élastiques sont pris en sandwich entre la feuille supplémentaire et la feuille de surface 3.

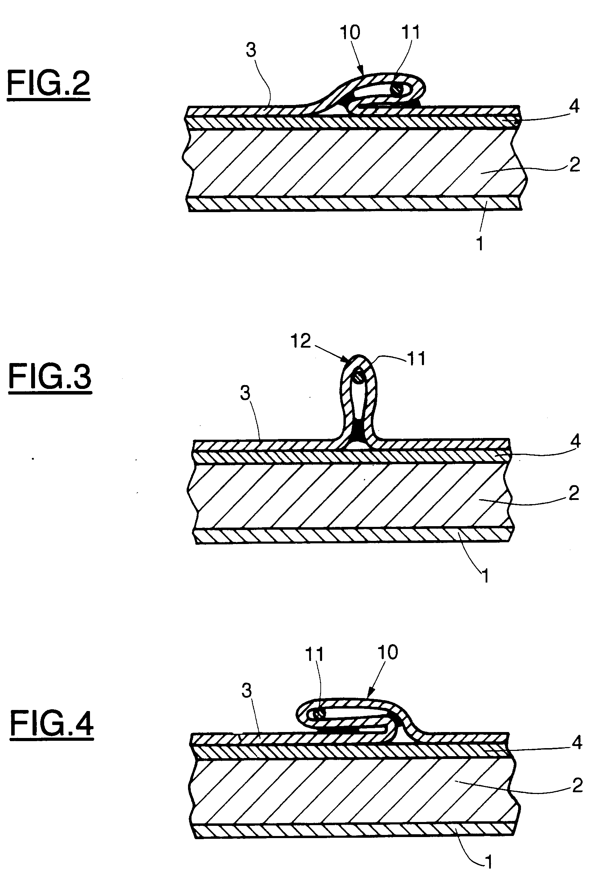

La feuille de surface 3 comporte également deux plis longitudinaux, parallèles 10, s'étendant sur toute la longueur de la couche-culotte et disposés de part et d'autre de l'ouverture longitudinale 5 à proximité des bords longitudinaux de celle-ci. Les bords inférieurs des plis 10 sont réunis par une ligne de fixation longitudinale, par exemple par une ligne de collage, sur toute la longueur des plis, pour former des gaines fermées. Des éléments élastiques longitudinaux 11 sont disposés à l'état tendu à l'intérieur des plis dans la partie supérieure des gaines fermées formées par ces plis 10. Chacun des plis 10 comprend en outre une première zone allant du bord transversal arrière 7 de la couche-culotte sensiblement jusqu'au bord transversal avant de l'ouverture 5 dans laquelle, comme le montre mieux la figure 2, le pli 10 est rabattu vers l'intérieur de la couche-culotte en direction de l'axe longitudinal médian XX' de la couche-culotte et est fixé à la partie sous-jacente de la feuille de surface intérieure 3 sur toute la longueur de cette première zone, par exemple par une ligne de collage. Chacun des plis 10 comporte également une seconde zone allant sensiblement du bord transversal avant de l'ouverture 5 jusqu'au bord transversal avant 6 de la coche-culotte dans laquelle le pli 10 est libre sur une majeure partie de cette seconde zone et est rabattu vers l'extérieur dans la direction opposée à l'axe longitudinal médian et fixé à la parie sous-jacente de la feuille de surface intérieure 3, par exemple par collage, à proximité du bord transversal avant 6 de la couche-culotte. Ainsi, comme on le voit mieux sur la figure 3, du fait que le bord supérieur du pli 10 est libre dans la majeure partie de cette seconde Zone, et que le pli 10 est fixé à la feuille de surface 3 sur la totalité de la longueur de la première zone et à proximité du bord transversal avant 6, en subissant une torsion de 180°, le pli 10 se relève dans la partie libre de cette seconde zone formant ainsi de petites barrières longitudinales 12, empêchant des fuites d'urine. De plus, cette fixation des plis 10 aux parties sous-jacentes de la feuille de surface 3 fait que les plis 10 s'écartent l'un de l'autre en direction du bord transversal avant 6 de la couche-culotte, offrant ainsi une plus grande surface d'absorption en avant de l'ouverture 5 ce qui assure une meilleure répartition des urines dans le coussin absorbant et par conséquent un meilleur confort à l'utilisateur.

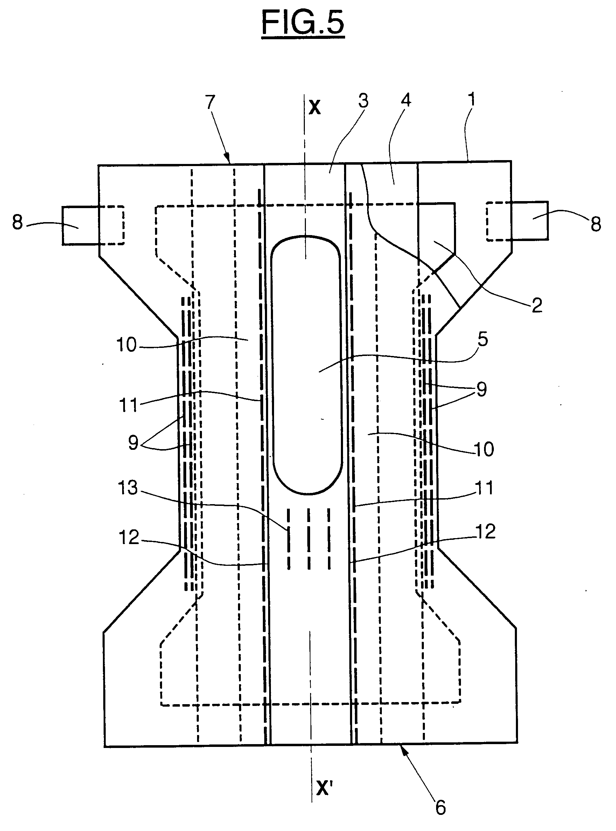

En se référant maintenant à la figure 5 on a représenté une autre réalisation d'une couche-culotte selon l'invention qui diffère de la couche-culotte de la figure 1 uniquement par le fait que les plis longitudinaux 10 sont rabattus dans leurs secondes zones vers l'intérieur en direction de l'axe longitudinal médian XX' et sont fixés aux parties sous-jacentes de la feuille de surface 3 près du bord transversal avant 6 de la couche-culotte. Ainsi, sous l'action des éléments élastiques longitudinaux 11, le bord libre des secondes zones des plis se soulèvent au dessus de la feuille de surface 3 sur la majeure partie de ces secondes zones formant ainsi des barrières latérales d'étanchéité vis à vis des fuites transversales d'urine.

On a réalisé ainsi une couche-culotte qui assure une excellente isolation des matières fécales vis-à-vis des urines et évite un contact prolongé entre celles-ci et la peau de l'utilisateur et qui en même temps permet une meilleure étanchéité vis-à-vis des fuites d'urine.

## Revendications

1. Article d'hygiène absorbant jetable, symétrique par rapport à un axe longitudinal médian (XX'), ayant des bords transversaux avant (6) et arrière (7), qui comprend une feuille extérieure 1 de support imperméable aux liquides, de forme générale rectangulaire, un coussin absorbant (2), de forme générale rectangulaire, fixé sur la feuille extérieure de support, une feuille de surface intérieure (3) perméable aux liquides recouvrant le coussin absorbant, fixée sur son pourtour à la feuille extérieure de support, ladite feuille de surface intérieure (3) comportant au moins une ouverture longitudinale (5) symétrique par rapport à l'axe longitudinal médian (XX') de la couche-culotte et de largeur inférieure à celle du coussin absorbant (2) cette ouverture longitudinale (5) étant décalée par rapport au centre de l'article, en direction du bord transversal arrière (7) de l'article, pour être disposée symétriquement par rapport à un point d'acquisition des selles situé à une distance prédéterminée dudit bord transversal arrière (7) de l'article, caractérisé en ce que : cette feuille de surface intérieure (3) est fixée au coussin absorbant à l'exception d'une zone située en-dessous de l'ouverture longitudinale (5) et de dimension supérieure à celle-ci, que deux plis longitudinaux (10) sensiblement parallèles à l'axe longitudinal médian (XX') de la couche-culotte, s'étendent sur toute la longueur de ladite couche-culotte de part et d'autre de l'ouverture longitudinale (5), chacun desdits plis (10) formant une gaine fermée dans laquelle est fixé, à l'état tendu, un élément élastique longitudinal (11),
et que chacun des plis (10) comporte une première zone longitudinale allant du bord transversal arrière (7) de l'article sensiblement jusqu'au bord transversal avant de l'ouverture longitudinale (5) dans laquelle le pli est rabattu vers l'intérieur en direction de l'axe longitudinal médian (XX') et est fixé à la partie sous-jacente de la feuille de surface intérieure (3) sur toute la longueur de ladite première zone, et une seconde zone allant dudit bord transversal avant de l'ouverture longitudinale (5) jusqu'au bord transversal avant (6) de l'article dans laquelle le pli (10) est libre sur une majeure partie de cette seconde zone et est rabattu soit vers l'extérieur dans la direction opposée à l'axe longitudinal médian (XX') soit vers l'intérieur en direction dudit axe et est fixé à la partie sous-jacente de la feuille de surface intérieure (3) à proximité du bord transversal avant (6) dudit article.

2. Article selon la revendication 1, caractérisé en ce que les plis (10) sont rabattus, dans leur seconde zone, vers l'extérieur' dans la direction opposée à l'axe longitudinal médian (XX').

3. Article selon la revendication 1 ou 2, caractérisé en ce que des éléments élastiques (13) sont fixés à l'état tendu, sensiblement symétriquement par rapport à l'axe longitudinal médian (XX') de l'article, à la feuille de surface intérieure (3), en avant de l'ouverture longitudinale (5).

4. Article selon la revendication 3, caractérisé en ce que les éléments élastiques (13) sont parallèles à l'axe longitudinal médian (XX').

5. Article selon la revendication 3, caractérisé en ce que les éléments élastiques (13) sont disposés en forme de V dont la pointe est dirigée vers le bord avant de l'ouverture (5).

6. Article selon l'une quelconque des revendications 1 à 5, caractérisé en ce qu'il comprend une feuille intermédiaire (4), perméable aux liquides, située entre le coussin absorbant (2) et la feuille de surface intérieure (3), la feuille de surface (3) étant fixée soit au coussin absorbant (2) soit à la feuille intermédiaire (4), à l'exception de ladite zone située en dessous de l'ouverture (5).

## Claims

1. Disposable absorbent article of hygien which is symmetrical with respect to a longitudinal mid axis (XX') having font (6) and rear (7) transverse edges, which comprises an outer support sheet 1 which is impermeable to liquids , of rectangular general shape, an absorbent pad (2) of rectangular general shape , fixed onto the outer support sheet, an inner surface sheet (3) which is permeable to liquids , covering the absorbent pad, fixed on its periphery to the outer support sheet, said inner surface sheet (3) comprising at least one longitudinal opening (5) which is symmetrical with respect to the longitudinal mid axis (XX') of the diaper and has a width less than that of the absorbent pad (2), said longitudinal opening (5) being offset with respect to the center of the article in the direction of the rear transverse edge (7) of the article, to be arranged symmetrically with respect to a point of acquisition of stools which is situated at a predetermined distance from said rear transverse edge (7) of the article,
characterized in that :
- this inner surface sheet (3) is fixed to the absorbent pad , with the exception of a region situated below the longitudinal opening (5) and of dimension greater that the latter, that two longitudinal folds (10) which are substantially parallel to the longitudinal mid axis (XX') of the diaper , extending over the entire length of said diaper on either side of the longitudinal opening (5), each of said folds (10) forming a closed sheath in which a longitudinal elastic element (11) is fixed, in the stretched state,
- and in that each of the folds (10) includes a first longitudinal region going from the rear transverse edge (7) of the article substantially as far as the front transverse edge of the longitudinal opening (5) into which the fold is folded toward the inside in the direction of the longitudinal mid axis (XX')and is fixed to the underlying part of the inner surface sheet (3) over the entire length of the said first region, and a second region going from transverse edge of the longitudinal opening (5) as far as the front transverse edge (6) of the article , in which the fold (10) is free over most of this second region and is folded either toward the outside in the direction opposite the longitudinal mid axis (XX') or toward the inside in the direction of said axis and is fixed to the underlying part of the inner surface sheet (3) in proximity to the font transverse edge (6) of said article.

2. Article according to claim 1 , characterized in that the folds (10) are folded, in their second region, toward the outside in the direction opposite the longitudinal mid axis (XX').

3. Article according to claim 1 or 2, characterized in that elastic elements (13) are fixed, in the stretched state, substantially symetrically with respect to the longitudinal mid axis (XX') of the article to the inner surface sheet (3), in front of the longitudinal opening 5.

4. Aricle according to claim 3, characterized in that the elastic elements (13) are parrallel to the longitudinal mid axis (XX').

5. Article according to claim 3, characterized in that the elastic elements (13) are arranged in a V shape whose tip points toward the front edge of the opening (5).

6. Article according to any one of claims 1 to 5 , characterized in that it comprises an intermediate sheet (4) which is permeable to liquids, situated between the absorbent pad (2) and the inner surface sheet (3), the surface sheet (3) being fixed either to the absorbent pad (2) or to the intermediate sheet (4), with the exception of said region situated below the opening (5).

## Patentansprüche

1. Wegwerfbarer absorbierender Hygieneartikel, der bezüglich einer Mittellängsachse (XX') symmetrisch ist und eine vordere (6) und eine hintere (7) Querkante besitzt und eine flüssigkeitsundurchlässige außenliegende Trägerfolie (1) von im allgemeinen rechteckiger Form, eine absorbierende Polsterung (2) von im allgemeinen rechteckiger Form, die auf der außenliegenden Trägerfolie befestigt ist, eine flüssigkeitsdurchlässige innere Deckfolie (3), welche die absorbierende Polsterung bedeckt und auf ihrem Umfang an der äußeren Trägerfolie befestigt ist, aufweist, wobei die innere Deckschicht (3) mindestens eine zur Mittellängsachse (XX') der Höschenwindel angeordnete Längsöffnung (5) aufweist, deren Breite kleiner ist als die Breite der absorbierenden Polsterung und die dabei bezüglich der Mitte des Artikels in Richtung der hinteren Querkante (7) des Artikels so versetzt ist, daß sie symmetrisch zu einem Punkt der Übernahme des Stuhls angeordnet ist, welcher sich in einem vorgegebenen Abstand von der hinteren Querkante (7) des Artikels befindet, **dadurch gekennzeichnet**, daß diese innere Deckfolie (3) an der absorbierenden Polsterung befestigt ist, mit Ausnahme eines Bereichs unter der Längsöffnung (5), der größer ist als diese, daß zwei im wesentlichen zur Mittellängsachse (XX') der Höschenwindel parallele Längsfalten (10) der Höschenwindel sich über die gesamte Länge der Höschenwindel zu beiden Seiten der Längsöffnung (5) erstrecken, wobei jede der Falten (10) eine geschlossene Schlauchhülle bildet, in welcher im gespannten Zustand ein in Längsrichtung verlaufendes elastisches Element (11) befestigt ist, und daß jede der Falten (10) einen ersten Längsbereich aufweist, der von der hinteren Querkante (7) des Artikels im wesentlichen bis zur vorderen Querkante der Längsöffnung (5) verläuft, in welcher die Falte nach innen in Richtung der Mittellängsachse (XX') umgeschlagen und an dem darunterliegenden Bereich der inneren Deckschicht (3) über die ganze Länge des ersten Bereichs befestigt ist, sowie einen zweiten Bereich, der von der vorderen Querkante der Längsöffnung (5) bis zur vorderen Querkante (6) des Artikels verläuft und in welchem die Falte (10) über einen größeren Abschnitt dieses zweiten Bereichs frei und entweder nach außen in der zur Mittellängsachse (XX') entgegengesetzten Richtung oder nach innen in Richtung der Achse umgeschlagen und an dem darunterliegenden Bereich der inneren Deckschicht (3) in der Nähe der vorderen Querkante (6) des Artikels umgeschlagen ist.

2. Artikel nach Anspruch 1, **dadurch gekennzeichnet**, daß die Falten (10) in ihrem zweiten Bereich nach außen in die zur Mittellängsachse (XX') entgegengesetzte Richtung umgeschlagen sind.

3. Artikel nach Ansprüche 1 oder 2, **dadurch gekennzeichnet**, daß im gespannten Zustand im wesentlichen symmetrisch zur Mittellängsachse (XX') des Artikels an der inneren Deckschicht (3) vor der Längsöffnung (5) elastische Elemente (13) befestigt sind.

4. Artikel nach Anspruch 3, **dadurch gekennzeichnet**, daß die elastischen Elemente (13) parallel zur Mittellängsachse (XX') verlaufen.

5. Artikel nach Anspruch 3, **dadurch gekennzeichnet**, daß die elastischen Elemente (13) V-förmig angeordnet sind und die Spitze des V dabei zur Vorderkante der Öffnung (5) gerichtet ist.

6. Artikel nach einem der Ansprüche 1 bis 5, **dadurch gekennzeichnet**, daß er eine flüssigkeitsdurchlässige Zwischenfolie (4) aufweist, welche zwischen der absorbierenden Polsterung (2) und der inneren Deckschicht (3) liegt, wobei die Deckschicht (3) entweder an der absorbierenden Polsterung (2) oder an der Zwischenfolie (4) befestigt ist, ausgenommen in dem Bereich, der unter der Öffnung (5) liegt.
